# EUROPÄISCHE PATENTSCHRIFT

(11) **EP 0 873 146 B1**
(45) Veröffentlichungstag und Bekanntmachung des Hinweises auf die Patenterteilung: **14.03.2001**
(21) Anmeldenummer: 96943058.6
(22) Anmeldetag: 07.12.1996
(51) Int. Cl.: A61M 1/00, A61C 17/08

(54) **Absaugvorrichtung für medizinische Zwecke**
Suction device for medical purposes
Dispositif d'aspiration pour usages médicaux

(30) Priorität: 14.12.1995 DE 19546664
(43) Veröffentlichungstag der Anmeldung: 28.10.1998
(73) Patentinhaber: Laabs, Walter, 26419 Grafschaft-Schortens (DE); Appel, Hans-Günter, Prof. Dr., D-26419 Schortens (DE)
(72) Erfinder: Laabs, Walter, 26419 Grafschaft-Schortens (DE); Appel, Hans-Günter, Prof. Dr., D-26419 Schortens (DE)
(74) Vertreter: Siewers, Gescha, Dr.
(86) Internationale Anmeldenummer: EP9605488
(87) Internationale Veröffentlichungsnummer: WO9721454

(56) Entgegenhaltungen:
- EP-A- 0 337 617
- DE-A- 4 002 373
- US-A- 4 589 869

## Beschreibung

Die Erfindung betrifft eine Absaugvorrichtung für medizinische Zwecke mit besonderer Eignung für den Einsatz in der Minimal-Invasiven-Chirurgie (MIC).

Bei Operationen müssen austretendes Blut, Gewebeflüssigkeit, Gewebefragmente, Knochensplitter und -mehl sowie Eiter, aber auch Spülflüssigkeiten wie Kochsalz- oder Ringerlösung vom Operationsfeld entfernt werden. Während man früher hierfür Mulltupfer verwendete, werden heutzutage Absaugvorrichtungen eingesetzt. In der Zahnmedizin müssen z.B. Speichel, Blut und Bohrreste abgesaugt werden. In der Intensivmedizin, Anästhesie und Endoskopie müssen Körperflüssigkeiten verschiedener Viskosität und Zusammensetzung abgesaugt werden (z.B. Schleim, Blut, Urin, Stuhl, Galle, Magensaft etc. auch Kontrastflüssigkeiten).

Absaugvorrichtungen für medizinischen Zwecke bestehen im allgemeinen aus einer fest installierten oder mobilen Vakuumpumpe, die an einen Unterdrucksammelbehälter angeschlossen ist, der seinerseits über einen Überlaufschutz mit einer sogenannten Sekretflasche verbunden ist, die wiederum ihrerseits über den Absaugschlauch mit der Absaugspitze in Verbindung steht. Es gibt kleinlumige oder großlumige Sauger, wobei man als großlumige Sauger solche bezeichnet, bei denen der Innendurchmesser größer als 4,9 mm ist. Diese Sauger können aus Kunststoff, aber auch aus Metall oder anderen Werkstoffen bestehen.

Wenn beim Absaugen Gewebe die Öffnung der Saugspitze des Absaugers vollständig bedeckt, bildet sich ein Unterdruck, der in der Größenordnung bis an den Pumpenunterdruck von 0,1 bar heranreichen kann. Dadurch wird Gewebe durch den Luftdruck in die Saugöffnung hineingedrückt, so daß traumatische Gewebeschädigungen entstehen können. Außerdem kann Gewebe am Innenrand der Saugöffnung abgeschert werden, was ebenfalls zu Verletzungen führen kann.

Absaugvorrichtungen für medizinische Zwecke sind bereits bekannt. Die DE-PS 40 02 373 beschreibt Absaugvorrichtungen für medizinische Zwecke mit einem Sauger aus Metall oder Kunststoff, der einen oder mehrere Seitenöffnungen mit einem auf Unterdruck ansprechenden Ventil besitzt. Patentgemäß sind die Seitenöffnungen als Langlöcher oder als durchgehende Nut ausgebildet und mit einer dünnen Schlitzfolie bzw. mit einer schlitzbaren Folie, die entweder als Schlauch aufziehbar oder aber als Folienstreifen aufklebbar ist, abgedeckt. Auf diese Weise läßt sich selbst bei Pumpenunterdrucken, die an 0,1 bar heranreichen, das Festsaugen der Saugspitze am Gewebe vermeiden. Sogenannte traumatische Gewebedefekte treten nicht mehr auf. Außerdem wird durch Variieren der Schlitzgröße eine optimale Anpassung der Saugleistung an die individuellen Gegebenheiten ermöglicht. Hierdurch wird gleichfalls eine bei Operationen als störend empfundene manuelle, durch zeitweiliges Abdecken der an der Saugspitze befindlichen Seitenlöcher bewirkte Vakuumkontrolle überflüssig. Gegenüber Saugern, die oberhalb der Saugspitze in üblicher Weise Seitenöffnungen aufweisen, können Absaugvorrichtungen nach DE 40 02 373 kontinuierlich ohne störende Nebengeräusche betrieben werden.

Obwohl die nach dem Stand der Technik bekannten Absaugvorrichtungen mittlerweile traumatische Gewebeverletzungen sowie störende Betriebsgeräusche weitgehend vermeiden helfen und insbesondere auch in der Neurochirurgie mit Erfolg eingesetzt werden, sind sie in ihrer Einsatzbreite konstruktionsbedingt auf solche Operationen beschränkt, die bei Atmosphärendruck stattfinden. Allerdings ist zu beobachten, daß in stetig zunehmender Zahl Operationen, wie z.B. im Bereich der Minimal-Invasiven-Chirurgie regelmäßig im Überdruckbereich von 0,01 bis 0,1 bar durchgeführt werden. Die Minimal-Invasive-Chirurgie gewinnt infolge rascher Fortschritte in der Medizintechnik sowie im opto-elektronischen Bereich zunehmend an Bedeutung. Dadurch, daß bei MIC-Operationen der operative Aufwand sehr gering gehalten werden kann und der tatsächliche chirurgische Eingriff auf ein Minimum reduziert wird, kann der Heilungsprozeß in der Regel beschleunigt und gleichzeitig die zumeist sehr kostenintensive stationäre Pflege deutlich verkürzt werden. Insbesondere der letztgenannte Aspekt wird aufgrund der gesellschaftspolitischen Bedeutung, die kostendämpfenden Maßnahmen im Gesundheitswesen mittlerweile beigemessen wird, zu einer forcierten Ausweitung der MIC-Operationstechniken und Anwendungen führen.

Bei der Minimal-Invasiven-Chirurgie ist es ebenfalls notwendig, Blut und Spülflüssigkeit während eines operativen Eingriffes über eine Absaugvorrichtung abzuführen. Um traumatische Gewebeverletzungen durch Festsaugen der Saugspitze zu vermeiden, sollte auch bei der Minimal-Invasiven-Chirurgie auf die vorteilhaften Eigenschaften einer Absaugvorrichtung nach DE 40 02 373 nicht verzichtet werden. MIC-Eingriffe werden in der Regel am Operationsbereich mit einem Überdruck von 0,01 bis 0,1 bar durchgeführt. Da die Seitenöffnungen der Absaugvorrichtung, um ihre patentgemäße Aufgabe erfüllen zu können, sich außerhalb des Körpers, d.h. im Normaldruckbereich befinden, bestünde, solange die Absaugvorrichtung nicht in Betrieb ist, bei den Schlitzfolien nach DE 40 02 373 ständig die Gefahr, daß Druckgas austritt.

Es besteht daher noch ein Bedarf an Absaugvorrichtungen, die auch im OP-Überdruckbereich und hier insbesondere bei MIC-Anwendungen einsetzbar sind, ohne gleichzeitig traumatische Gewebeverletzungen und unerwünschte Geräuschkulissen zu verursachen und die darüber hinaus einfach aufgebaut und bei Bedarf ieicht zu reinigen und zu sterilisieren sind. Aufgabe der Erfindung ist somit, die Vorteile der Absaugvorrichtung nach DE 40 02 373 ebenfalls für bei Überdruck durchgeführte Operationen und insbesondere für solche der Minimal-Invasiven-Chirurgie aufrechtzuerhalten.

Diese Aufgabe wird mit einer Absaugvorrichtung gemäß Anspruch 1 gelöst.

Insbesondere werden nun Absaugvorrichtungen mit einem Sauger aus Kunststoff oder Metall vorgeschlagen, die eine oder mehrere Seitenöffnungen mit einem auf Unterdruck ansprechenden Ventil besitzen und dadurch gekennzeichnet sind, daß die Seitenöffnungen mit sich mittig überlappenden Folienhälften abgedeckt sind, von denen die eine aus hochelastischem dünnen, die andere aus steifem unflexiblen Material besteht. Wichtig ist die Elastizität der beiden Materialien, während die Stärke der Folien keine wesentliche Rolle spielt und daher auch gleichstarke Folien eingesetzt werden können. Erfindungswesentlich ist weiterhin, daß die steife Folienhälfte die flexible Folienhälte im Überlappungsbereich überdeckt. Vorteilhafterweise dekken die Folienstreifen von gegenüberliegenden Seiten aus jeweils mehr als die Hälfte der Seitenlochoberfläche ab. Die geradkantigen Folienstreifen können dabei derart auf der Saugeroberfläche befestigt werden, daß die überlappenden Folienkanten parallel zueinander verlaufen und der Überlappungsbereich bei Langlöchern entlang der Mittelachse verläuft.

Die Seitenöffnungen können als Langlöcher ausgebildet sein und befinden sich in der Regel in einem großen Abstand von der Saugerspitze. Dadurch wird sichergestellt, daß die Ventile beim Eintauchen der Saugerspitze in den Überdruckbereich außerhalb des Körpers bzw. im Normaldruckbereich verbleiben können.

Auf diese Weise ist sichergestellt, daß ein kritischer Unterdruck, der zu traumatischen Gewebeverletzungen führen könnte, vermieden wird. Gleichzeitig ist gewährleistet, daß, sobald sich im Saugrohr der nicht in Betrieb befindlichen Absaugvorrichtung ein Überdruck bildet, der flexible Folienstreifen gegen den überlappenden, nicht nachgebenden steifen Folienstreifen gedrückt und das Saugrohr verschlossen wird. Ein Austritt von Druckgas findet nicht statt. Demzufolge können konstante Operationsbedingungen gewährleistet werden und der Sauger kann permanent im Operationsbereich verbleiben. Es ist nicht notwendig, die Saugerspitze je nach Bedarf und Notwendigkeit von außen an das Operationsfeld heranzuführen bzw. zu entfernen.

Die Erfindung wird im folgenden anhand der Zeichnungen näher erläutert:
- Fig. 1: zeigt einen erfindungsgemäßen Sauger in Seitenansicht.
- Fig. 2: zeigt denselben Sauger im Querschnitt bei Überdruck im Saugrohr.
- Fig. 3: zeigt denselben Sauger im Querschnitt bei Unterschreiten eines kritischen Unterdruckes.
- Fig. 4: zeigt die Aufsicht auf ein mit Folienstreifen abgedecktes Langloch.

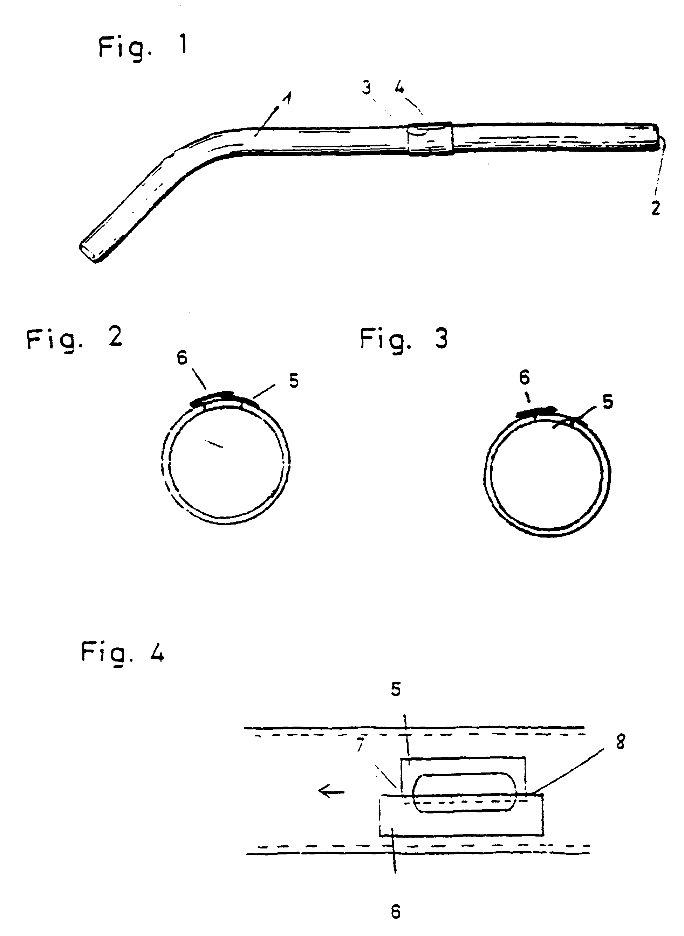

Der Sauger (1) verfügt über eine Öffnung (2) und über ein oder mehrere Langlöcher (3), die ihrerseits mit sich mittig überlappenden Folien abgedeckt sind, wobei die eine (5) aus flexiblem Material, die andere (6) aus steifem Material besteht. Die Folien (5, 6) sind derart angeordnet, daß im Überlappungsbereich die steife Folie (6) die flexible Folie (5) überdeckt. Aus der Aufsichtzeichnung (Fig. 4) ergibt sich, daß die sich überlappenden Folienkanten (7) und (8) parallel zur Längsachse des Langloches verlaufen, die Folienbahnen jeweils etwas mehr als die Hälfte bis zu ca. 2/3 der Fläche des Langloches bedecken und der sich überlappende Bereich sich ungefähr im Bereich der Mittelachse des Langloches befindet. Die benutzten Folien bestehen vorteilhafterweise aus flexiblen bzw. steifen Kunststoffolienmaterialien und lassen sich unproblematisch auf dem Saugrohr anbringen.

## Patentansprüche

1. Absaugvorrichtung für medizinische Zwecke mit besonderer Eignung für den Einsatz in der Minimal-Invasiven-Chirugie (MIC), die einen als Saugrohr ausgebildeten Sauger mit mindestens einer Saugöffnung (2) sowie mit einer oder mehreren Seitenöffnungen (3) aufweist, wobei die Seitenöffnungen (3) jeweils mit einem auf Unterdruck ansprechenden Ventil (4) versehen sind, dadurch gekennzeichnet, daß die Seitenöffnungen (3) mit sich mittig überlappenden Folienhälfen (5, 6) abgedeckt sind, von denen die eine (5) aus hochelastischem dünnen Material, die andere (6) aus steifem unflexiblen Material besteht, und wobei die steife Folie (6) im Überlappungsbereich die flexible Folie (5) überdeckt.

2. Absaugvorichtung nach Anspruch 1, dadurch gekennzeichnet, daß die Seitenöffnungen (3) als Langlöcher ausgebildet sind.

3. Absaugvorrichtung nach Anspruch 1 oder 2, dadurch gekennzeichnet, daß die Seitenöffnungen im Bereich normalen Druckes positioniert sind.

4. Absaugvorrichtung nach Anspruch 1 bis 3, dadurch gekennzeichnet, daß die flexiblen und steifen Folienteile (5, 6) von gegenüberliegenden Seiten aus jeweils mehr als die Hälfte der Seitenlochoberfläche abdecken.

5. Absaugvorrichtung nach Anspruch 1 bis 4, dadurch gekennzeichnet, daß die überlappenden Folienkanten (7, 8) parallel zueinander verlaufen.

6. Absaugvorrichtung nach Anspruch 1 bis 5, dadurch gekennzeichnet, daß die Folienteile (5) und (6) aus Kunststoffmaterial bestehen.

## Claims

1. A suction device for medical purposes with particular suitability for use in minimally invasive surgery (MIS), which has an aspirator, in the form of a suction tube, with at least one suction opening (2) and with one or more lateral openings (3), the lateral openings (3) each being provided with a valve (4) which reacts to pressure below atmospheric, characterised in that the lateral openings (3) are covered by centrally overlapping sheet-halves (5, 6), of which one (5) thereof is composed of highly-resilient thin material and the other (6) thereof of rigid inflexible material, and the rigid sheet (6) covering the flexible sheet (5) in the region of overlap.

2. A suction device according to Claim 1, characterised in that the lateral openings (3) are in the form of oblong holes.

3. A suction device according to Claim 1 or 2, characterised in that the lateral openings are positioned in the normal-pressure region.

4. A suction device according to Claims 1 to 3, characterised in that the flexible and rigid sheet-parts (5, 6) each cover, from opposite sides, more than half of the lateral-hole surface.

5. A suction device according to Claims 1 to 4, characterised in that the overlapping sheet-edges (7, 8) run parallel to one another.

6. A suction device according to Claims 1 to 5, characterised in that the sheet parts (5) and (6) are composed of plastics material.

## Revendications

1. Dispositif d'aspiration à usage médical, convenant en particulier pour une utilisation en chirurgie invasive minimale, comportant un aspirateur réalisé sous forme de tube d'aspiration avec au moins un orifice d'aspiration (2) ainsi qu'un ou plusieurs orifices latéraux (3), les orifices latéraux (3) étant chacun équipés d'une soupape (4) réagissant à une dépression, **caractérisé** en ce que les orifices latéraux (3) sont recouverts par des moitiés de feuilles (5, 6) qui se chevauchent au milieu, dont l'une (5) est en un matériau mince et hautement élastique tandis que l'autre (6) est en un matériau rigide et non flexible, la feuille (6) rigide recouvrant la feuille (5) flexible dans la zone de chevauchement.

2. Dispositif d'aspiration selon la revendication 1, **caractérisé** en ce que les orifices latéraux (3) sont réalisés sous forme de trous oblongs.

3. Dispositif d'aspiration selon la revendication 1 ou 2, **caractérisé** en ce que les orifices latéraux sont positionnés dans une zone de pression normale.

4. Dispositif d'aspiration selon les revendications 1 à 3, **caractérisé** en ce que les parties de feuille flexible et rigide (5, 6) recouvrent respectivement plus de la moitié de la surface des orifices latéraux, à partir de côtés opposés.

5. Dispositif d'aspiration selon les revendications 1 à 4, **caractérisé** en ce que les bords de feuille (7, 8) qui se chevauchent sont mutuellement parallèles.

6. Dispositif d'aspiration selon les revendications 1 à 5, **caractérisé** en ce que les parties de feuille (5) et (6) sont en une matière plastique.
